(19) Europäisches Patentamt

European Patent Office

Office européen des brevets

(11) **EP 3 424 316 A1**

(12) **EUROPEAN PATENT APPLICATION**
published in accordance with Art. 153(4) EPC

(43) Date of publication:
**09.01.2019 Bulletin 2019/02**

(21) Application number: **17760030.1**

(22) Date of filing: **28.02.2017**

(51) Int Cl.:
*A01K 67/027* (2006.01)     *A61K 45/00* (2006.01)
*A61P 25/28* (2006.01)      *G01N 33/15* (2006.01)
*G01N 33/50* (2006.01)      *C12N 15/09* (2006.01)

(86) International application number:
**PCT/JP2017/007955**

(87) International publication number:
**WO 2017/150566 (08.09.2017 Gazette 2017/36)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **01.03.2016 JP 2016039349**

(71) Applicant: **SMC Global Asset, Inc.
Tokyo 144-0035 (JP)**

(72) Inventor: **TAKANO, Hirofumi
Tokyo 144-0035 (JP)**

(74) Representative: **Arends, William Gerrit
Marks & Clerk LLP
90 Long Acre
London WC2E 9RA (GB)**

(54) **NON-HUMAN ANIMAL HAVING DYSTROPHY CAUSED BY PROTEIN AGGREGATION**

(57)     The present invention provides a model animal for establishing an effective therapy for a protein aggregation disease typified by Alzheimer's disease and the like.

More specifically, the present invention provides the followings:

(A) a non-human animal that exhibits a degenerative symptom attributed to protein aggregation, wherein the degenerative symptom attributed to protein aggregation is induced by misfolding of the protein and said degenerative symptom is promoted; and

(B) a method for producing the non-human animal that exhibits the degenerative symptom attributed to the protein aggregation, comprising the following (1) and (2):

(1) inducing misfolding of the protein to cause the degenerative symptom attributed to the protein aggregation in the non-human animal, and

(2) giving a treatment to promote the degenerative symptom attributed to the protein aggregation in the non-human animal.

EP 3 424 316 A1

**Description**

TECHNICAL FIELD

The present invention relates to non-human animals exhibiting degenerative symptoms attributed to protein aggregation.

BACKGROUND ART

A protein aggregation disease (PAD) is known as a disease concept that mainly exhibits degenerative diseases due to abnormal accumulation of a pathologic protein such as amyloid and the like originated from misfolding of a protein based on a mutation. However in recent years, a wide-ranging disease concept including abnormal aggregation of a protein based on the misfolding due to not only a mutation but also a toxic stimulation to a cell and aging has been recognized and diversified into those even referred to as a syndrome that become extremely important in an aged society.
The prior art that disclosed findings that can be associated with PAD includes the followings.
Non-Patent Literature 1 discloses that 2-amino-1-methyl-6-phenyl-1H-imidazo[4,5-b]pyridine (PhIP) has toxicity in a dopaminergic neuron selective manner in a rat primary culturedd cell.
Non-Patent Literature 2 discloses an intracerebroventricular (icv) streptozocin (STZ) model as an animal model for Alzheimer's type dementia having a diabetes like background.
Non-Patent Literature 3 discloses an adriamycin nephropathy (AN) model as a model for chronic kidney disease.
Non-Patent Literature 4 discloses a doxorubicin inducible model as a model for cardiac fibrosis.
Non-Patent Literature 5 discloses a diethyl nitrosamine (DEN)-carbon tetrachloride (CCL4) inducible model as a hepatic cancer model.

PRIOR ART REFERENCES

PATENT LITERARURES

[NON-PATENT LITERATURES]

[NON-PATENT LITERATURE 1]
Griggs A. M. et al., Toxicological Science, 2014, 140(1): 179-189.
[NON-PATENT LITERATURE 2]
Singh,Birdavinder, et al., Journal of Renin-Angiotensin-Aldosterone System 14. 2(2013): 124-136.
[NON-PATENT LITERATURE 3]
Resolvin D1 Protects Podocytes in Adriamycin-induced Nephropathy through Modulation of 14-3-3β Acetylation, Xueming Zhang, et al, Plos one_2013, 8_1-17
[NON-PATENT LITERATURE 4]
Hagir B. Suliman et al., J Clin Invest., 2007, 117, 12, 3730-3741
[NON-PATENT LITERATURE 5]
Hagir B. Suliman et al., J Clin Invest., 2007, 117, 12, 3730-3741

DISCLOSURE OF INVENTION

PROBLEM TO BE SOLVED BY THE INVENTION

However, no effective therapy for PAD typified by Alzheimer's disease and the like has been established as of this moment, and studies on such disease models are important. Conventional disease models are mainly models obtained by genetic modification to cause an intracellular mutation, and these have contributed insufficiently to establishment of sufficient therapies. Additionally, no model by which extracellular aggregation can be sufficiently analyzed has been available, and thus, it is difficult to develop a new medication for new PADs that have appeared in the aged society.
As a result of an extensive study for solving the above problems, the present inventors have found that a good model animal for PAD can be produced by inducing a misfolding of a protein in a non-human animal to cause a degenerative symptom attributed to protein aggregation in the non-human animal, followed by treating the non-human animal to promote this degenerative symptom, and have completed the present invention.

MEANS FOR SOLVING PROBLEM

That is, the present inventions are as follows.

[1] A non-human animal that exhibits a degenerative symptom attributed to protein aggregation, wherein the degenerative symptom attributed to the protein aggregation is induced by misfolding of the protein and said degenerative symptom is promoted.

[2] The non-human animal according to [1], wherein the degenerative symptom attributed to the protein aggregation is induced by a chemical substance having cytotoxicity.

[3] The non-human animal according to [2], wherein the chemical substance having the cytotoxicity is (1) combination of diethyl nitrosamine and carbon tetrachloride, (2) 2-amino-1-methyl-6-phenyl-1H-imidazo[4,5-b] pyridine, (3) streptozotocin, (4) adriamycin, or (5) doxorubicin.

[4] The non-human animal according to any of [1] to [3], wherein said degenerative symptom is promoted by inducing generation of lipid peroxide.

[5] The non-human animal according to [4], wherein the generation of said lipid peroxide is induced by feeding of a high fat diet.

[6] The non-human animal according to any of [1] to [5], wherein said non-human animal is a model animal of the degenerative disease attributed to the protein aggregation selected from the group consisting of (1) hepatic cancer, (2) Parkinson's disease, (3) Alzheimer's disease, (4) chronic kidney disease, and (5) cardiac fibrosis.

[7] The non-human animal according to any of [1] to [6], wherein said non-human animal is a model animal of the degenerative disease attributed to the protein aggregation selected from the group consisting of the following:

(1) a model animal of hepatic cancer produced by a combination of diethyl nitrosamine and carbon tetrachloride, and feeding of the high fat diet;
(2) a model animal of Parkinson's disease produced by 2-amino-1-methyl-6-phenyl-1H-imidazo[4,5-b]pyridine and feeding of the high fat diet;
(3) a model animal of Alzheimer's disease produced by streptozotocin and feeding of the high fat diet;
(4) a model animal of chronic kidney disease produced by adriamycin and feeding of the high fat diet; and
(5) a model animal of cardiac fibrosis produced by doxorubicin and feeding of the high fat diet.

[8] The non-human animal according to any of [1] to [7], wherein said non-human animal is a rodent.
[9] The non-human animal according to [8], wherein the rodent is a mouse.
[10] A method for producing a non-human animal that exhibits a degenerative symptom attributed to protein aggregation, comprising the following (1) and (2):

(1) inducing misfolding of a protein to cause the degenerative symptom attributed to the protein aggregation in the non-human animal, and
(2) giving a treatment to promote the degenerative symptom attributed to the protein aggregation in the non-human animal.

[11] A non-human animal that exhibits a degenerative symptom attributed to protein aggregation, and to which 2-amino-1-methyl-6-phenyl-1H-imidazo[4,5-b]pyridine is administered.

[12] A method for producing a non-human animal that exhibits a degenerative symptom attributed to protein aggregation, comprising administering 2-amino-1-methyl-6-phenyl-1H-imidazo[4,5-b]pyridine.

[13] A method for screening of a preventive or therapeutic drug for a protein aggregation disease, comprising the following (a) to (c):

(a) administering a test substance to the non-human animal according to any of [1] to [9] and [11];
(b) evaluating whether the test substance has or not an action of improving a degenerative symptom attributed to protein aggregation; and
(c) selecting the test substance having the action of improving the degenerative symptom attributed to the protein aggregation as the preventive or therapeutic drug for the protein aggregation disease.

[14] A method for evaluating a side effect of a test medicine comprising the following (a) and (b):

(a) administrating the test medicine to the non-human animal according to any of [1] to [9] and [11]; and
(b) evaluating whether the test medicine has or not an action upon a degenerative symptom attributed to protein aggregation.

[15] A biological sample prepared from the non-human animal according to any of [1] to [9] and [11] and including a degenerated site attributed to protein aggregation.

EFFECT OF THE INVENTION

The non-human animal of the present invention can be used for preclinical trials of various therapeutic drugs as a model animal of a protein aggregation disease. Also, the present invention is useful for searching a target in drug development and therapeutic targets.

BRIEF DESCRIPTION OF DRAWINGS

Fig. 1-1 shows TH positive cell counts in brain in Vehicle group (1), PhIP group (2) and PhIP + HFD (high fat diet) group (3), and Fig. 1-2 shows photographs showing TH immunostaining in the brain in Vehicle group (1), PhIP group (2) and PhIP + HFD group (3).
Fig. 2 shows photographs showing Lewy bodies in the brain in Vehicle group (1), PhIP group (2) and PhIP + HFD group (3).
Fig. 3 shows results of a Y maze test (alternation rate (%)) in Sham control group (1), STZ group (2) and STZ + HFD group (3).
Fig. 4 shows photographs showing results of collagen type IV immunostaining in the brain in Sham control group (1), Intracerebroventricular (icv) STZ group (2), and Intracerebroventricular STZ + HFD group (3).
Fig. 5-1 shows a rate (%) of a Sirius red staining positive area on a kidney section in Normal group (1), Adriamycin group (2), and Adriamycin + HFD group (3), and Fig. 5-2 shows photographs showing Sirius red staining on the kidney section in Normal group (1), Adriamycin group (2), and Adriamycin + HFD group (3)
Fig. 6-1 shows a rate (%) of an immunostaining positive area on a kidney section in Normal group (1), Adriamycin group (2), and Adriamycin + HFD group (3), and Fig. 6-2 shows photographs showing F4/80 immunostaining on the kidney section in Normal group (1), Adriamycin group (2), and Adriamycin + HFD group (3).
Fig. 7-1 shows a rate (%) of a Sirius red staining positive area on a heart section in Vehicle group (1), Vehicle + HFD group (2), Doxorubicin group (3) and Doxorubicin + HFD group (4), and Fig. 7-2 shows photographs showing Sirius red staining on the heart section in Vehicle group (1), Vehicle + HFD group (2), Doxorubicin group (3) and Doxorubicin + HFD group (4).
Fig. 8-1 shows the number of tumors in liver in Normal group (1), DEN-CCl4 group (2), and DEN-CCl4 + HFD group (3), and Fig. 8-2 shows a tumor size in the liver in Normal group (1), DEN-CCl4 group (2), and DEN-CCl4 + HFD group (3).
Fig. 9-1 shows a rate (%) of a Sirius red staining positive area on a liver section in Normal group (1), DEN-CCl4 group (2), and DEN-CCl4 + HFD group (3), and Fig. 9-2 shows photographs showing Sirius red staining on the liver section in Normal group (1), DEN-CCl4 group (2), and DEN-CCl4 + HFD group (3)
Fig. 10-1 shows a rate (%) of an immunostaining positive area on a liver section in Normal group (1), DEN-CCl4 group (2), and DEN-CCl4 + HFD group (3), and Fig. 10-2 shows photographs showing p53 immunostaining on the liver section in Normal group (1), DEN-CCl4 group (2), and DEN-CCl4 + HFD group (3).

EMBODIMENTS FOR CARRYING OUT THE INVENTION

The present invention provides a non-human animal that exhibits a degenerative symptom attributed to protein aggregation. In the non-human animal of the present invention, the degenerative symptom attributed to the protein aggregation is caused by induction of misfolding of the protein, and said degenerative symptom is promoted.
The non-human animal used in the present invention is not particularly limited as long as it is typically an animal generally used as an experimental animal, and is preferably a vertebrate. Examples of the vertebrate include birds (e.g., chicken) and mammalian animals, but are preferably the mammalian animals. The mammalian animals include, for example, rodents (e.g., mice, rats, guinea pigs, rabbits), primates (e.g., monkeys, chimpanzees), dogs, cats, pigs, cattle and sheep. A genetic background of an animal used for producing the non-human animal of the present invention is not particularly limited, and an animal having any genetic background can be utilized. Typically, a wild type animal can suitably be used.
The non-human animal of the present invention is useful as a model animal of a protein aggregation disease (PAD). PAD includes, for example, neurodegenerative diseases (e.g., Alzheimer's disease, Parkinson's disease), kidney diseases (e.g., chronic kidney disease), cardiac diseases (e.g., cardiac fibrosis), hepatic diseases (hepatic cancer), pulmonary diseases (e.g., COPD), ocular diseases (e.g., retinitis pigmentosa, cataract, cornea amyloidosis, cornea dystrophy, glaucoma, oculopharyngeal muscular dystrophy), muscular diseases (e.g., Inclusion body myositis), hair diseases (e.g., scalp hypotrichosis), vascular diseases (e.g., arteriosclerosis), gastrointestinal diseases (e.g., Hirschsprung's disease), cutaneous diseases (e.g., cutaneous amyloidosis), peripheral nerve disorders (e.g., Charcot-Marie disease), pituitary diseases (e.g., abnormal hormone due to aged pituitary), menopausal disorders, cancers, fibrosis, and pancreatic diseases (e.g., type II diabetes).

Aggregated proteins in PAD above include, for example, α-synuclein, β-amyloid, tau, collagen, p53, SP-C, rhodopsin, crystalline, lactoferrin, keratoepithelin, PAPB2, APP, Aβ, corneodesmosin, ApoB100/LDL, RET, myelin protein 22/0, prolactin, and p53.

Sites at which the aggregated protein deposits include, for example, brain (e.g., substantia nigra compacta, ventral tegmental area, hippocampus, cerebral cortex), kidney (e.g., stroma), heart (e.g., ventricular endomysium), liver, lung, eyes (e.g., retina, crystalline lens, cornea, aqueous humor body, oculopharyngeal muscle), muscles, hair follicles, blood vessels, gastrointestinal tract (e.g., stomach, small intestine, large intestine), skin, peripheral nerves, pituitary gland, and pancreas. The sites at which the aggregated protein deposits also include, for example, intracellular sites (e.g., in nuclei, in cytoplasm) and extracellular sites.

Methods for detecting the aggregated protein in the non-human animal of the present invention include, for example, any staining methods such as an antibody staining, Sirius red staining and the like.

The non-human animal of the present invention can be produced by the following (1) and (2):

(1) inducing misfolding of a protein in a non-human animal to cause a degenerative symptom attributed to protein aggregation; and
(2) treating the non-human animal to promote the degenerative symptom attributed to the protein aggregation.

In the non-human animal of the present invention, the degenerative symptom attributed to the protein aggregation can be caused by administering a substance having an ability to induce the misfolding of the protein. The substance having the ability to induce the misfolding of the protein includes, for example, a substance that elicits the misfolding of the protein and a substance that inhibits an action of a factor that eliminates or reduces aggregation of a misfolded protein. Specifically, examples of such a substance include a chemical substance having cytotoxicity, a chaperon inhibitory substance, an autophagy inhibitory substance, and an endoplasmic reticulum-associated decomposition inhibitory substance.

The chemical substance having the cytotoxicity includes, for example, 2-amino-1-methyl-6-phenyl-1H-imidazo[4,5-b]pyridine, streptozotocin, doxorubicin, adriamycin, diethyl nitrosamine, and carbon tetrachloride.

The chaperon inhibitory substance includes, for example, tunicamycin, ETB, dithiothreitol, β-mercaptoethanol, and thapsigargin.

The autophagy inhibitory substance includes, for example, Bafilomycin A1, Chloroquine, E-64d, LY-294002, LY 303511, Pepstatin A, and (+)-Wortmannin.

The endoplasmic reticulum-associated decomposition inhibitory substance includes, for example, Bortezonib, MG-132, Carfilzomib, MLN9708, Ixazomib, PI-1840, ONX-0914, Oprozomib, CEP-18770, Nafamostat, Aspirin, Gabexate, PR-619, P5091, IU1, TCID, LDN-57444, Degrasyn, ML323, P22077, p97 Inhibitors, NMS-873, DBeQ, MNS, NSC697923, BAY 11-7082, PYR-41, Thalidomide, TAME, RITA, Tenovin-1, E3 Ligase Activators, (-)-Parthenolide, NSC 207895, Nutlin-3, and JNJ-26854165.

An administration form of the substance having the ability to induce the misfolding of the protein is not particularly limited, and includes, for example, subcutaneous administration (e.g., subcutaneous injection), intravenous administration, intracerebroventricular administration, oral administration, intraperitoneal administration, and the like.

The substance having the ability to induce the misfolding of the protein can be administered in any amount capable of inducing the misfolding of an objective protein depending on a type of the substance.

The substance having the ability to induce the misfolding of the protein can appropriately be administered to an animal at any age (e.g., age between one day of age and 20 weeks, 15 weeks or 10 weeks of age) depending on types of the substance and the animal.

A treatment to promote the degenerative symptom attributed to the protein aggregation can be performed by a different treatment from induction of the misfolding of the protein. For example, this treatment can be carried out by inducing generation of lipid peroxide. The induction of the generation of lipid peroxide can be performed by giving a diet or a substance that induces the generation of lipid peroxide. The diet or the substance that induces the generation of lipid peroxide includes, for example, a high fat diet, an atherosclerotic diet, an iron-containing diet, streptozotocin, carbon tetrachloride, and a combination thereof.

As used herein, the high fat diet refers to a fat sample containing 20% or more (preferably 30% or more) oil and fat content. In the high fat diet, a percentage of a calorie derived from the oils and fats occupied in total calories can be about 40 % or more (preferably 50% or more). The oils and fats include, for example, cholesterol, unsaturated fatty acids (e.g., linoleic acid), and saturated fatty acids. Ingredients to be combined with the high fat diet includes, for example, powdered beef fat, milk casein, egg white powder, L-cystine, safflower oil, crystalline cellulose, maltodextrin, lactose, sucrose, and the like. Feeds commercially available as feeds for animals can generally be utilized as the high fat diet. The high fat diets include, for example, High Fat Diet 32 (CLEA, Japan Inc.) and D12492 (Research Diet, Inc.) that are commercially available as animal feeds for research.

The treatment for promoting the degenerative symptom may be carried out simultaneously with the induction of the misfolding of the protein. Alternatively, it may be carried out before or after inducing the misfolding of the protein. Alternatively, the treatment may be carried out from before or simultaneously with the induction to after the induction.

In preferable embodiments, the non-human animal of the present invention exhibits a degenerative symptom in a central nervous system attributed to aggregation of α-synuclein, where the degenerative symptom of the central nervous system (substantia nigra compacta, ventral tegmental area) attributed to aggregation of α-synuclein is induced by 2-amino-1-methyl-6-phenyl-1H-imidazo[4,5-b]pyridine (PhIP), and the degenerative symptom is promoted by inducing the generation of lipid peroxide. Such a non-human animal is useful as a Parkinson's disease model. Such a non-human animal can be produced, for example, by administering PhIP in an amount (e.g., 0.5 to 50 mg/kg) capable of causing α-synuclein aggregation to an animal at 4 to 15 weeks of age (preferably 5 to 9 weeks of age) and simultaneously or subsequently inducing the generation of lipid peroxide (e.g., feeding a high fat diet).

In another preferable embodiment, the non-human animal of the present invention exhibits a degenerative symptom in the central nervous system attributed to β-amyloid aggregation and/or tau aggregation, where the degenerative symptom in the central nervous system (hippocampus, cerebral cortex) attributed to the β-amyloid aggregation and/or tau aggregation is induced by an initial dosing of streptozocin, and the degenerative symptom is promoted by subsequent administration of streptozocin. Such a non-human animal is useful as an Alzheimer's disease model. Such a non-human animal can be produced, for example, by administering streptozocin in an amount capable of causing the β-amyloid aggregation and/or tau aggregation (e.g., 0.3 to 30 mg/kg) to an animal at one day to 13 weeks of age (preferably one day to 10 weeks of age), and subsequently administering streptozocin in an amount capable of inducing the generation of lipid peroxide (e.g., 0.1 to 10 mg/kg).

In another preferable embodiment, the non-human animal of the present invention exhibits a degenerative symptom in kidney (stroma) attributed to collagen aggregation, where the degenerative symptom in kidney (stroma) attributed to the collagen aggregation is induced by adriamycin, and the degenerative symptom is promoted by inducing the generation of lipid peroxide. Such a non-human animal is useful as a model of chronic kidney diseases. Such a non-human animal can be produced, for example, by administering adriamycin in an amount capable of causing the collagen aggregation (e.g., 1 to 50 mg/kg) to an animal at 4 to 15 weeks of age (preferably 6 to 10 weeks of age), and simultaneously or subsequently inducing the generation of lipid peroxide (e.g., feeding the high fat diet).

In another preferable embodiment, the non-human animal of the present invention exhibits a degenerative symptom in the heart (ventricular endomysium, stroma) attributed to collagen aggregation, where the degenerative symptom in the heart (ventricular endomysium, stroma) attributed to the collagen aggregation is induced by doxorubicin, and the degenerative symptom is promoted by inducing the generation of lipid peroxide. Such a non-human animal is useful as a cardiac fibrosis model. Such a non-human animal can be produced, for example, by administering doxorubicin capable of causing the collagen aggregation in the heart (e.g., 1 to 75 mg/kg) to an animal at 4 to 15 weeks of age (preferably 6 to 10 weeks of age), and simultaneously or subsequently inducing the generation of lipid peroxide (e.g., feeding the high fat diet).

In another preferable embodiment, the non-human animal of the present invention exhibits a hepatic cancer attributed to collagen aggregation, where the hepatic cancer attributed to hepatic disorder and/or hepatic fibrosis is induced by DEN-CCL4, and carcinogenesis is promoted by inducing the generation of lipid peroxide. Such a non-human animal is useful as a hepatic cancer model. Such a non-human animal can be produced, for example by administering DEN (e.g., 0.1 to 10 mg/kg) and CCl4 (e.g., 0.02 to 1 mL/kg) in amounts capable of causing the hepatic cancer to an animal at 1 to 50 weeks of age, and simultaneously or subsequently inducing the generation of lipid peroxide (e.g., feeding the high fat diet).

In the production of the non-human animal of the present invention, when 2-amino-1-methyl-6-phenyl-1H-imidazo[4,5-b]pyridine is used as the substance having the ability to induce the misfolding of the protein, the treatment for promoting the degenerative symptom may be omitted. The present invention also provides the non-human animal produced by such a method.

The present invention also provides a method for screening of a preventive or therapeutic drug for the protein aggregation disease.

The method for screening of the present invention comprises the following steps (a) to (c):

(a) administering a test substance to the non-human animal of the present invention;
(b) evaluating whether the test substance has or not an action to improve the degenerative symptom attributed to the protein aggregation; and
(c) selecting the test substance having the action to improve the degenerative symptom attributed to the protein aggregation as a preventive or therapeutic drug for the protein aggregation disease.

In step (a), the test substance is not particular limited, and includes, for example, a single compound such as a natural compound, an organic compound, an inorganic compound, a protein, a peptide, and the like. Also the test substance further includes an expressed product from a chemical library or a genetic library, a cell extract, a cell culture supernatant, a fermentation microbial product, a marine organism extract, a plant extract, and the like.

A method for administering the test substance is not particularly limited, and for example, the test substance can be administered orally or via injection. When the test substance is a protein, a viral vector having a gene encoding the protein is constructed, and taking advantage of its infectious capacity, the gene can also be introduced into the non-human animal of the present invention.

In step (b), the evaluation whether the test substance has or not the action to improve the degenerative symptom attributed to the protein aggregation can be performed by evaluating whether the protein aggregation is inhibited or not in the non-human animal, or whether the degenerative symptom attributed to the protein aggregation is alleviated or not. The evaluation for inhibition of the protein aggregation can be performed by any staining method such as antibody staining, Sirius red staining, and the like. The evaluation for alleviation of the degenerative symptom attributed to the protein aggregation can appropriately be performed, for example, depending on observations for the degenerative symptom (e.g., pathological observations, behavioral observations).

In step (c), the test substance that has the action to improve the degenerative symptom attributed to the protein aggregation is selected as the preventive or therapeutic drug for the protein aggregation disease.

The present invention also provides a method for evaluating a side effect of a test medicine. The method for evaluation of the present invention is useful for development pf pharmaceuticals in which an action (e.g., side effect) upon the degenerative symptom attributed to the protein aggregation is not desired and which exhibit predetermined drug efficacy (e.g., pharmaceuticals with reduced side effects).

The method for evaluation of the present invention comprises the following (a) and (b):

(a) administering a test medicine to the non-human animal of the present invention; and
(b) evaluating whether the test medicine has or not an action upon the degenerative symptom attributed to the protein aggregation.

In step (a), the test substance is not particularly limited as long as it is a substance exhibiting a predetermined drug efficacy (e.g., medicine, physiologically active substance), and for example, the aforementioned test substance can be used.

In step (b), the evaluation whether the test medicine has or not the action upon the degenerative symptom attributed to the protein aggregation can be performed in the same manner as in (b) in the method for screening of the present invention.

The present invention also provides a biological sample prepared from the non-human animal of the present invention and including a degenerated site attributed to the protein aggregation.

The biological sample of the present invention comprises the degenerated site attributed to the protein aggregation. The degenerated site attributed to the protein aggregation is a deposit site of the aggregated protein as aforementioned. The degenerated site attributed to the protein aggregation may comprise a substance having an ability to induce the misfolding of the protein that caused the protein aggregation, in addition to the aggregated protein. A combination of such a protein and the substance is as described above. The sample includes, for example, an entire organ, an organ section, and a primary cultured cell. The biological sample of the present invention is useful for, for example, screening for preventive or therapeutic drugs for the protein aggregation disease.

[EXAMPLES]

Hereinafter, the present invention will be described in detail by Examples, but the present invention is not limited to these Examples.

Example 1: Development of novel Parkinson's disease model utilizing dopamine neural toxicity of 2-amino-1-methyl-6-phenyl-1H-imidazo[4,5-b]pyridine (PhIP)

[Study objective]

Parkinson's disease is a neural disease caused by degeneration of dopaminergic nerve in the central nervous system and exhibiting bradykinesia and akinesia often in combination with dementia. The number of patients is said to be about 10 million, and there are many existing drugs, but a phenomenon referred to as wearing off where a duration of drug action becomes short with long term use of the drug has become a problem (see Reference 1).

On the other hand, in non-clinical efforts, a chemical inducible Parkinson's model utilizing cytotoxicity of a dopamine analog such as 6-OHDA or MPTP, and a gene modification model in Parkin or PINK1 known as a risk gene have been utilized, but no model that sufficiently reflects characteristics of both pathology and symptoms have been developed so far (see Reference 2). In particular, a model having a pathological feature where the dopaminergic nerve are killed progressively has been expected.

In recent years, it has been reported that 2-amino-1-methyl-6-phenyl-1H-imidazo[4,5-b]pyridine (PhIP) exhibits toxicity in a dopamine nerve selective manner in rat primary cultured cells. PhIP is one type of cyclic amine known as a carcinogenic substance. Cyclic amine is contained in cocked meats, and an association between ingestion of meats and Parkinson's disease has been pointed out by epidemiologic surveys (see Reference 3).

Thus, in the present Example, an effect of PhIP administration on mice *in vivo* was examined in an effort to produce a novel mouse model of Parkinson's disease utilizing PhIP. A dosage concentration of PhIP was determined to be 0.4 mg/mL (see Reference 4), PhIP was administered via a tail vein, and mice were sacrificed on 28 days after the administration. In order to examine a synergistic effect by combination of load with a high fat diet (HFD), a group receiving a dosing of PhIP at a concentration of 0.2 mg/mL and the load with HFD was set, and mice were sacrificed on 28 days after the administration. Dopaminergic cell counts and Lewy body counts in SNc and VTA were measured by observing and evaluating TH (tyrosine hydroxylase) immunostaining and the Lewy bodies to confirm cytotoxicity of the dopaminergic nerves and aggregation of $\alpha$-synuclein.

[Criteria to be preserved and guidelines to be complied, and the like]

Animal welfare

"The Act on Welfare and Management of Animals"

(Article 105 on October 1, 1973, Article 105 finally revised on August 30, 2011)

"The Guideline for Raising and Keeping of Laboratory Animals"

(The Ministry of Environment Notification No. 88, April 28, 2006)

"Fundamental Guideline for Proper Conduct of Animal Experiment and Related Activities in Academic Research Institutes"

(The Ministry of Education, Culture, Sports, Science and Technology Notification No. 71, June 1, 2006).

The present experiments were approved by the Animal Experiment Committee at Stelic MC (SMC Laboratories, Inc.), and performed according to the Guideline for Management and Welfare of Experimental Animals mandated in the experimental facility (Regulations for Animal Experiments at Stelic MC).

[Experimental materials and methods]

Test substance and solvent

Test substance

Name: PhIP (2-amino-1-methyl-6-phenyl-1H-imidazo[4,5-b]pyridine)

Solvent for test substance

Name: DMSO + 0.9% saline (20:80 v/v)

Animals to be used

Twelve male C57BL/6J mice at 7 weeks of age were purchased from Japan SLC, Inc., put into quarantine, and habituated and raised. After habituation, the mice were confirmed to have no problem in their general state, and then subjected to the study.

Feed

For an HFD loading group, a high fat diet (solid feed containing about 32% fat and oil and having calorie where a percentage of a calorie from the fat and oil occupied in total calories is about 57%) was fed freely from an animal arrival day to a sacrifice day.

Grouping

The twelve male mice were grouped into three groups by a body weight stratified random sampling method so that average body weights were equal on the day before starting the study (starting the administration of PhIP).

Route, period and frequency of administration

Administration via the tail vain was selected for administering DMSO and PhIP at each concentration, and a frequency of the administration was once on Day 0.

Group constitution

A group constitution is shown in Table 1.

Table 1. Group constitution

| Test groups | Administration amount (mg/kg) | Concentration (mg/mL) | Administration volume (mL/kg) | Sex | The number of animals | Animal number |
|---|---|---|---|---|---|---|
| Vehicle administration group | 0 | 0 | 5 | Male | 4 | 101-104 |
| PhIP (0.4 mg/mL) administration group | 1.0 | 0.4 | 5 | Male | 4 | 201-204 |
| PhIP (0.4 mg/mL) + HFD load group | 1.0 | 0.4 | 5 | Male | 4 | 301-304 |

Observation of general states, measurement of body weights, and measurement of feed intakes

During the period of the experiment, the general state was observed, the body weights were measured, and feed intakes were measured. For reckoning of an experimental day, the following is applied.

Day of stating PhIP administration: Day 0

Observation of general states

This was performed before measuring the body weights. The general states such as an outer surface of a body, a nutrient state, an attitude, behaviors and excretions were observed for all of the individuals.

Necropsy

On Day 28, all animals planned to be necropsied were anesthetized with isoflurane, sacrificed by beheading, and brain was harvested. A part of about one-third from a front of the harvested brain was coronally cut and discarded. A part of two-thirds from a backward was fixed in 4% PFA, which was replaced with 30% sucrose solution, and stored at -80°C as an OTC embedded frozen block.

Immunohistochemical staining

Preparation of frozen sections

The frozen block was sliced into a thickness of 10 μm using a cryostat, and a section was attached on a slide glass. The section was obtained so that both SNc and VTA could be observed simultaneously on a cross-section. The cross-section was determined with reference to ALLEN Brain Atlas. The frozen sections were subjected to TH immunostaining.

TH immunostaining and measurement of positive cells

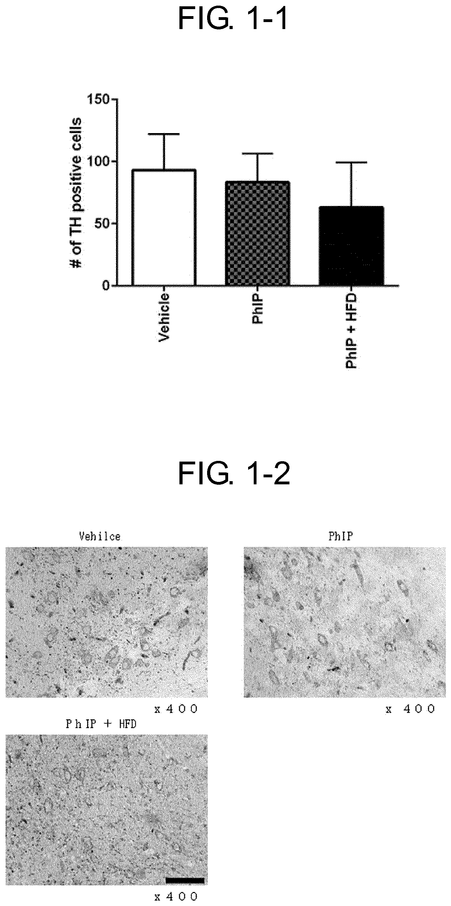

The frozen section was treated with 0.03% hydrogen peroxide in water at ambient temperature for 10 minutes to inhibit endogenous peroxidase. The section was washed with PBS, and subsequently reacted with an anti-TH antibody (Abcam) at 4°C overnight. The section was washed with PBS, and reacted with a peroxidase-labeled anti-rabbit antibody (Jackson, IR) diluted 25 times at ambient temperature for 30 minutes. The section was washed with PBS, and fixed with 1% glutaraldehyde solution at ambient temperature for one minute. Color was developed by a chromogenic substrate (Simple Stain DAB, NICHIREI Corporation). After developing the color, the section was washed with RO water and fixed with 1% glutaraldehyde solution. The section was passed through hematoxylin diluted 8 times for one minute, immediately washed with RO water, and mounted with Aquatex (Merck KGaA). The preparation was observed using a bright field microscope. Photographs of SNc and VTA regions were taken using a CCD camera. The number of TH positive cells in each field were counted and an average value of the TH positive cell counts per area was calculated. As a result, decrease of the TH positive cell counts was observed in PhIP + HFD group compared with PhIP group. Thus, decrease of dopaminergic nerves due to the load of HFD was observed (Fig. 1-1, 1-2).

HE staining and measurement of Lewy bodies

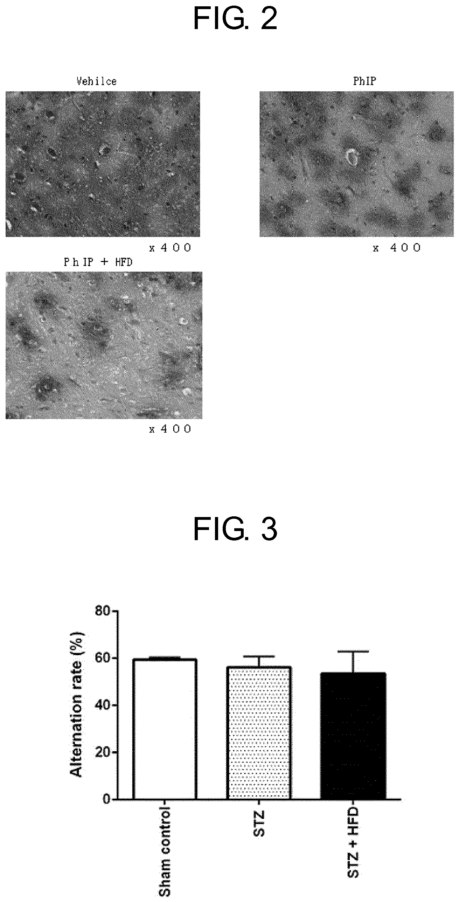

The frozen section was penetrated in a Lily Mayer hematoxylin solution (Muto Pure Chemicals Co. Ltd.) for 10 minutes. An extra staining solution was washed off using RO water, a color was controlled by running water for 10 minutes, subsequently the section was immersed in 1% eosin Y ethanol aqueous solution (Wako Pure Chemical Industries Ltd.), and immersed in RO water. The stained section was dehydrated and cleared by alcohol series of 70% to 100% and xylene, and subsequently mounted with Entellan new (Merck KGaA) to subject to observation. The preparation was observed using the bright field microscope (Leica Microsystems). As a result, increase of Lewy bodies was observed compared in PhIP + HFD group with PhIP group, suggesting increase of $\alpha$-synuclein aggregation due to HFD load (Fig. 2).

Flow of statistical analysis

Tests of difference between means in respective groups were carried out by Student t-test using Prism 6 (GraphPad Software Inc.), and the PhIP group at each concentration was compared with the vehicle group.
A representative value was expressed as mean $\pm$ SD, and the test was carried out using a significance level of 5%.

[References]

1. Pahwa R. et al., Current medical research and opinion, 2009, 25(4): 841-849.
2. Blesa J. et al., NioMed Research Internatinal, 2012, 2012.
3. Griggs A. M. et al., Toxicological Science, 2014, 140(1): 179-189.
4. Teunissen S.F. et al., Journal of Chromatography B, 2010, 878(25): 2353-2362.

Example 2: Non-clinical evaluation study for Alzheimer's disease using icv STZ

[Study objective]

Alzheimer's disease is classified into familial Alzheimer's that develops by genetic factors and sporadic Alzheimer's that develops by environmental factors. 99% of patients with Alzheimer's disease is said to be sporadic, but many of gene modified model mice mimic pathology and onset of familial Alzheimer's (see Reference 5). Therefore, development of a model animal for sporadic Alzheimer's disease that does not rely on gene modification has been required. Further, it has been demonstrated by the epidemiologic survey that about 60% of patients with diabetes are associated with dementia. Thus, Alzheimer's disease is also referred to as the third diabetes (see Reference 6).
An icv STZ model is a model animal of Alzheimer's type dementia having a diabetes like background, which occurs by impairing an intracerebral insulin signal in mice having a background of diabetes. Compared with a gene modified model, this icv STZ alone model (see Reference 7) exhibits intense inflammation and neural function disorder, and thus, pathological onset and pathological progress are thought to be caused by different mechanism from that in the gene modified model. However, no aggregation of a pathologic protein such as amyloid and tau associated with neural cell death was observed, and this model was difficult to be said to be a model close to clinical symptoms. This time, mice having deposition of a pathologic protein in cerebral tissue is reproduced by using mice where diabetes was induced by HFD. Use of this model potentially provides new finding for therapeutic drug candidates that cannot sufficiently be evaluated by the gene modified model.

[Criteria to be preserved and guidelines to be complied, and the like]

Animal welfare

"The Act on Welfare and Management of Animals"

(Article 105 on October 1, 1973, Article 105 finally revised on August 30, 2011)

"The Guideline for Raising and Keeping of Laboratory Animals"

(The Ministry of Environment Notification No. 88, April 28,

2006) "Fundamental Guideline for Proper Conduct of Animal Experiment and Related Activities in Academic Research Institutes"

(The Ministry of Education, Culture, Sports, Science and Technology Notification No. 71, June 1, 2006).

The present experiments were approved by the Animal Experiment Committee at Stelic MC (SMC Laboratories, Inc.), and performed according to the Guideline for Management and Welfare of Experimental Animals mandated in the experimental facility (Regulations for Animal Experiments at Stelic MC).

[Experimental materials and methods]

Animals to be used

Test animals

Thirty male SFP mice (C57BL/6J, Japan SLC, Inc.) at 6 weeks of age were purchased, put into quarantine, and habituated and raised.

Grouping

Using the body weight on the day before starting the administration as an indicator, the mice were grouped into three groups (10 mice/group) so that average body weights became even. A group constitution is shown in Table 2.

Table 2. Group constitution

| Test groups | Sex | The number of animals | Animal number |
|---|---|---|---|
| Sham group | Male | 2 | 101-102 |
| icv STZ group | Male | 2 | 201-202 |
| icv STZ + HFD group | Male | 2 | 301-302 |

Production of model mouse

A model was induced by administering streptozotocin (each 3 μL) to an intracerebroventricular space of a mouse at 7 weeks of age on a day of staring the study (Day 0) and Day 2. The administration was performed once in both right and left locations (from the left).

Observation of general states, measurement of body weights, and measurement of feed intakes

During the period of the experiment, the general state was observed, the body weights were measured, and feed intakes were measured. For reckoning of an experimental day, the following is applied.

Day of starting icv-STZ administration at 7 weeks of age: 0 day (Day 0)

Observation of general states

This was performed before measuring the body weights. The general states such as an outer surface of a body, a nutrient state, an attitude, behaviors and excretions were observed for all of the individuals.

Behavioral experiment

Analysis of spontaneous alternation behavior (SAB) by Y-maze test (Reference 8).
Habituation in a Y-maze was performed 24 hours before performing a behavioral experiment (after completing the administration on Day 26). That is, a mouse was placed in a center of the maze and allows to search freely for 5 minutes.
After completing the final administration on Day 27, one trial was performed as a test. The mouse was placed in the center of the maze, and allowed to search for 8 minutes. During this period, a frequency of an event where the mouse entered arms and an order of entries into the arm were entirely recorded. After 8 minutes elapsed, the mouse was brought back into a home cage, and the Y-maze was disinfected with 50% ethanol to perform a subsequent mouse test.
An alternation rate (%) was used as an indicator of SAB. The alternation rate was calculated by the following formula 1.

```
100 × (Frequency of entries into different arm three times
in a row) / (Total frequency of entries into arm) (Formula
1)
```

As a result, decrease of the alternation rate was observed in Icv-STZ + HFD group compared with Icv-STZ group (Fig. 3).

Necropsy

On Day 28, after measuring a blood glucose level using a life checker, all animals planned to be necropsied were anesthetized with diethyl ether, sacrificed by beheading, and the brain was harvested. The harvested brain was weighed and cut into halves in a sagittal plane. A right brain part was fixed with 4% PFA (4°C, 2h), which was replaced with a 30% sucrose solution (4°C, O/N). On the following day, it was embedded with OTC and frozen-stored (-80°C). A left brain part was weighed, and then rapidly frozen and stored (-80°C) .

Immunohistochemical staining

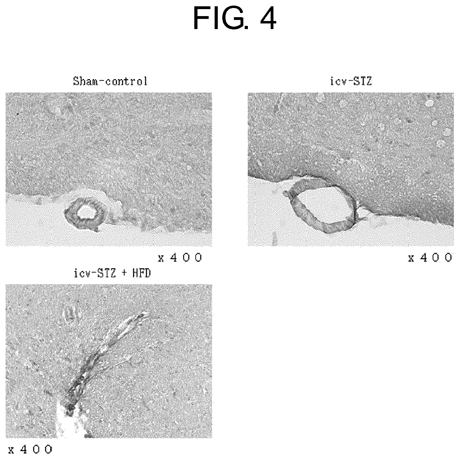

Immunostaining for collagen type IV was performed on frozen sections. As a result, intense staining was observed in a circumference of cerebral blood vessels to confirm increased accumulation of collagen type IV in Icv-STZ + HFD group compared with Icv-STZ group (Fig. 4).

Production of sections

Sections having a thickness of 10 $\mu$m were made using the cryostat at -20°C.

[References]

5. Kamboh, M. I., et al. "Genome-wide association study of Alzheimer's disease." Translational psychiatry 2.5(2012): e117.

6. Arvanitakis, Zoe, et al. "Diabetes mellitus and risk of Alzheimer disease and decline in cognitive function." Archives of neurology 61. 5(2004): 661-666.

7. Mehan, Sidharth, et al. Dementia-A Complete Literature Review on Various Mechanisms Involves in Pathogenesis and an Intracerebroventricular Streptozotocin Induced Alzheimer's Disease., INTECH Open Access Publisher, 2012.

8. Gorrie, George H., et al. "Dendritic spinopathy in transgenic mice expressing ALS/dementia-linked mutant UBQLN2." Proceedings of the National Academy of Sciences 111. 40(2014): 14524-14529.

Example 3: Development of model animal for chronic kidney disease

[Study objective]

Chronic kidney disease (CKD) is an inclusive term of pathology where renal functions are chronically reduced by primary disease such as diabetes and hypertension, and becomes a risk of terminal renal failure and cardiovascular diseases (see Reference 9). The number of patients with CKD has increased year by year, and patients with terminal renal failure who requires artificial dialysis also have increased (see Reference 9). No fundamental therapeutic drug for CKD is available at present, and it is thought to be important to suppress progressive kidney disorders such as protein urea, glomerular hypertension, glomerular disorder and renal tubular stromal disorder that commonly appear when the primary disease progresses (see Reference 10) .

Unilateral ureteral obstruction (UUO) model, 5/6 nephrectomy (5/6 Nx) model and adriamycin nephropathy model (AN) are available as the models of chronic kidney disease. Characteristics and possible endpoints of each model are shown in Table 3, but any models seems to be far from a model reflecting the clinical symptoms. This time, as a new model animal for developing a therapeutic drug for CKD, an AN administration model mouse was investigated. In order to examine a synergistic effect by combination with the load of high fat diet (same as in Example 1), a group receiving AN administration and HFD load was set, and raised and sacrificed in the same manner.

Table 3. Comparison of respective animals

| Test groups | | UUO | 5/6Nx | AN |
|---|---|---|---|---|
| Strain | | C57BL/6 | 129/SV | BALB/C, C57BL6 |
| Preparation method | | Ureteral obstruction | Nephrectomy | Adriamycin i.v. |
| Test Period | | 2 weeks | 12 weeks | 4 weeks |
| Evaluation item | Urea protein | - | ++ | ++ |
| | Urea albumin | - | ++ | ++ |
| | Plasma creatinine | - | ++ | ++ |
| | Glomerular disorder | - | ++ | ++ |
| | renal tubular stromal fibrosis | ++ | + | ++ |
| | Cardiac hypertrophy/ fibrosis | - | - | + |
| References | | 11 | 12 | 13 |

[Criteria to be preserved and guidelines to be complied, and the like]

Animal welfare

"The Act on Welfare and Management of Animals"

(Article 105 on October 1, 1973, Article 105 finally revised on August 30, 2011)

"The Guideline for Raising and Keeping of Laboratory Animals"

(The Ministry of Environment Notification No. 88, April 28, 2006)

"Fundamental Guideline for Proper Conduct of Animal Experiment and Related Activities in Academic Research Institutes"

(The Ministry of Education, Culture, Sports, Science and Technology Notification No. 71, June 1, 2006).

The present experiments were approved by the Animal Experiment Committee at Stelic MC (SMC Laboratories, Inc.), and performed according to the Guideline for Management and Welfare of Experimental Animals mandated in the experimental facility (Regulations for Animal Experiments at Stelic MC).

[Experimental materials and methods]

Reagents

Adriamycin (Doxorubicin hydrochloride)

Name: Doxorubicin hydrochloride

Manufacturer: Wako Pure Chemical Industries Ltd.

Animals to be used

Species (strain): C57BL/6
Sex: male
Weeks of age at arrival: 8 weeks of age
Basis of setting weeks of age: set based on Reference 13 where the AN model was produced.
Grade: SPF
Supplier: Japan SLC Inc.

Group constitution

The group constitution is shown in Table 4.

Table 4. Group constitution

| Test groups | Concentration of adriamycin administered | Sex | The number of animals |
|---|---|---|---|
| Normal | - | Male | 4 |
| Adriamycin | 25 mg/kg | Male | 10 |
| Adriamycin | 25 mg/kg | Male | 2 |

Production of model mouse

A test animal was held in a restraint device, adriamycin was dissolved in saline (0.9% saline) at a concentration in Table 4, and administered at 10 mL/kg via a tail vein. In a normal group, 10 mL/kg of saline was administered via the tail vain. A method of production referenced Reference 13 in which the AN model was produced.

Observation of general states and measurement of body weight

During the experimental period, the general states were observed, and the body weight was measured. When a dead animal was found, it was immediately necropsied. For reckoning of an experimental day, the following is applied. Adriamycin dosing day: 0 day (Day 0)

Observation of general states

This was performed before measuring the body weights. The general states such as an outer surface of a body, a nutrient state, an attitude, behaviors and excretions were observed for all of the individuals.

Necropsy

Each necropsy animal was sacrificed by heart puncture for blood sampling, and kidney was harvested. The harvested kidney was immersed in Bouin fixation solution (Sigma-Aldrich Japan) and fixed at room temperature for 24 hours, followed by being embedded with paraffin. A central piece was frozen in liquid nitrogen to use for gene analysis, and a lower piece was fixed with 4% paraformaldehyde-PBS for 5 hours, then immersed in 20% sucrose-PBS, embedded with OCT-compound, frozen in liquid nitrogen, and stored at -80°C.

Tissue analysis of kidney

Sirius red staining

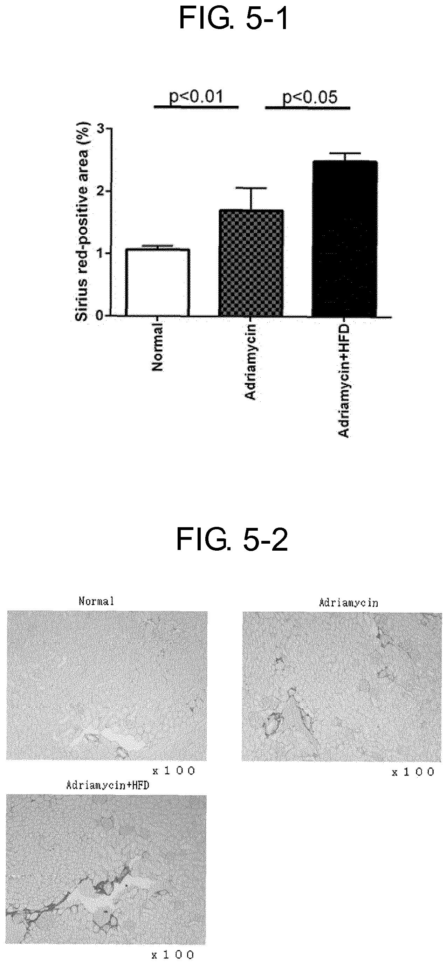

A paraffin section was deparaffinized and hydrophilized, followed by being immersed in 0.03% Picro-Sirius Red solution (Sirius Red: Waldeck) for 60 minutes. After passing through 0.5% acetic acid solution and RO water, the section was mounted with Entellan new (Merck KGaA). The preparation was observed using a bright field microscope (Leica Microsystems). A taken area in each field and a Sirius red positive area were measured based on images taken using a CCD camera (Leica Microsystems) using ImageJ software (National Institute of Health). As a result, a rate of a Sirius red positive area significantly increased and increase of collagen accumulation due to the HFD load was observed in Adriamycin + HFD group compared with Adriamycin group (Fig. 5-1, 5-2).

F4/80 immunostaining

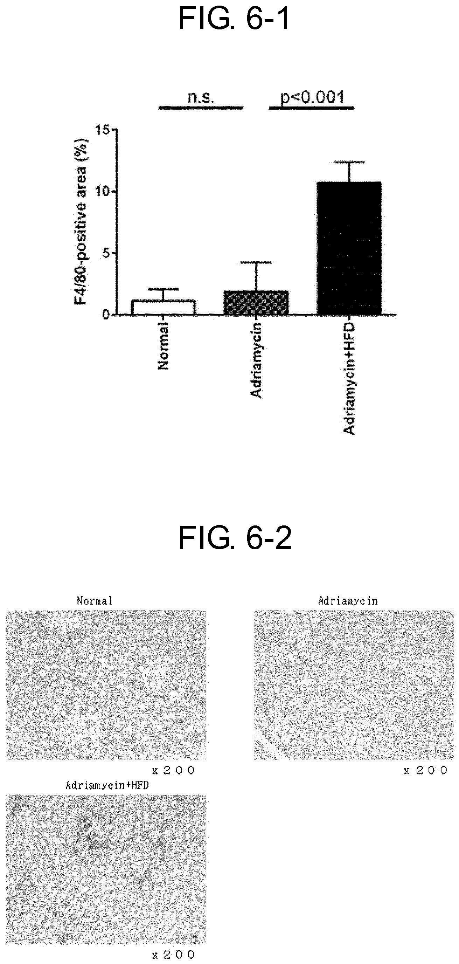

A frozen section made from the frozen block was fixed with acetone, and subsequently endogenous peroxidase was inhibited by 0.03% hydrogen peroxide in water. The section was reacted with a blocking solution (Blockace, DS Pharmabiomedical) at ambient temperature for 10 minutes, and then reacted with an anti F4/80 antibody (BMAbiomedical) diluted 200 times at 4°C overnight. After washing with PBS, the section was reacted with a peroxidase-labeled anti-rabbit IgG goat antibody (Invitrogen) diluted 25 times at ambient temperature for 30 minutes. A color was developed by a chromogenic substrate (Simple stain DAB, Nichirei Bioscience), and the section was mounted with Aquatex (Merck KGaA). The preparation was observed using the bright field microscope (Leica Microsystems). Glomerulus was photographed using the CCD camera (Leica Microsystems). A photographed area and an F4/80 positive area were measured based on obtained images using ImageJ software (National Institute of Health). As a result, a rate of F4/80 immunostaining positive area significantly increased and increased infiltration of inflammatory cells into glomerulus due to the HFD load was observed in Adriamycin + HFD group compared with Adriamycin group (Fig. 6-1, 6-2).
The rate of F4/80 positive area (inflammation area) (%) was calculated based on images photographed from the preparation using the bright field microscope, using ImageJ.

Flow of statistical analysis

Tests of difference between means in respective groups were carried out by Student t-test using Prism 4 (GraphPad Software Inc.)to compare between the normal group and the adriamycin dosing group and between the adriamycin dosing group and the adriamycin + HFD dosing group.
An average in each group was expressed as mean $\pm$ SD, and the test was carried out using a significance level of 5%.

[References]

9. Guide for CKD diagnosis 2009 edited by Japanese Society of Nephrology, Tokyo Igakusha.
10. Junji Takayama, A simple method for producing a model for reduction of renal functions in rats and mice. Folia Pharmacol. Jpn., 131: 37-42, 2008.
11. Models of chronic kidney disease, Hai-chun yang, et al, Drug discov Today Dis models_2010, 7_13-19
12. Model of robust induction glomerulosclerosis in mice: Importance of genetic background, Li-jun Ma, et al, Kidney International_2003, 64_350-355
13. Resolvin D1 Protects Podocytes in Adriamycin-induced Nephropathy through Modulation of 14-4-4beta Acetylation, Xueming Zhang, et al, Plos one_2013, 8_1-17

Example 4: Fibrosis evaluation study using high fat diet-loading cardiac fibrosis model

[Experimental background and objective]

Cardiac inflammation and fibrosis are deeply associated with cardiac failure and myocardial infarction (see Reference 14). No effective therapy has been established for cardiac diseases, and elucidation of mechanisms for cardiac inflammation and fibrosis as well as development of therapeutics have been desired.

A doxorubicin inducible model has been reported as a cardiac fibrosis model (see Reference 15). This time, a high fat diet was loaded to the doxorubicin inducible model to examine its effect on progress of the cardiac fibrosis.

[Criteria to be preserved and guidelines to be complied, and the like]

Animal welfare

"The Act on Welfare and Management of Animals"

(Article 105 on October 1, 1973, Article 105 finally revised on August 30, 2011)

"The Guideline for Raising and Keeping of Laboratory Animals"

(The Ministry of Environment Notification No. 88, April 28, 2006)

"Fundamental Guideline for Proper Conduct of Animal Experiment and Related Activities in Academic Research Institutes"

(The Ministry of Education, Culture, Sports, Science and Technology Notification No. 71, June 1, 2006).

The present experiments were approved by the Animal Experiment Committee at Stelic MC (SMC Laboratories, Inc.), and performed according to the Guideline for Management and Welfare of Experimental Animals mandated in the experimental facility (Regulations for Animal Experiments at Stelic MC).

[Experimental materials and methods]

Test substance

   Name: doxorubicin (doxorubicin hydrochloride, Sigma-Aldrich)

   Storage method: refrigerated (2 to 8°C)

Preparation of dosing solution

Preparation of doxorubicin dosing solution

   Preparation method: Doxorubicin was dissolved in saline at a concentration of 3 mg/mL.
   Preparation frequency: freshly prepared before dosing.
   Storage method: room temperature

Animals to be used

Test animals

   Species (strain): C57BL/6

Sex: male
Weeks of age at arrival: 9 weeks of age
Basis of setting weeks of age: set based on Reference 15 where the cardiac fibrosis model was produced.
Grade: SPF
Supplier: Japan SLC Inc.

Grouping

Using the body weight on the day before starting the administration as an indicator, the mice were grouped into four groups (6 mice/group) so that average body weights became even. The group constitution is shown in Table 5.

Table 5. Group constitution

| Test groups | Administration amount (mg/kg) | Concentration (mg/mL) | Administration volume (mL/kg) | Sex | The number of animal | Animal number |
|---|---|---|---|---|---|---|
| Vehicle group | - | - | 5 | Male | 6 | 101-106 |
| Vehicle + HFD group | - | - | 5 | Male | 6 | 201-206 |
| Doxorubicin group | 15 | 3 | 5 | Male | 6 | 301-306 |
| Doxorubicin + HFD group | 15 | 3 | 5 | Male | 6 | 401-406 |

Production of cardiac fibrosis model mouse

Doxorubicin was dissolved in saline at a concentration of 3 mg/mL, and it was intraperitoneally administered at a dosage of 5 mL/kg in C57BL/6 mice at 10 weeks of age using Myshot. In Vehicle group, 5 mL/kg of saline was administered intraperitoneally. A production method was set based on Reference 16 in which the cardiac fibrosis model mouse was produced.
During an experimental period, the high fat diet (same as in Example 1) was fed freely in Vehicle + HFD group and Doxorubicin + HFD group.

Observation of general states and measurement of body weights

During the experimental period, the general states were observed, and the body weight was measured. When a dead animal was found, it was immediately necropsied. For reckoning of an experimental day, the following is applied.
Day of starting production of cardiac fibrosis model mice: 0 day (Day 0)

Observation of general states

This was performed before measuring the body weights. The general states such as an outer surface of a body, a nutrient state, an attitude, behaviors and excretions were observed for all of the individuals.

Necropsy

On Day 28, all of the mice planned to be necropsied were sacrificed by exsanguination from abdominal aorta, and the heart was harvested. The harvested heart was divided into two at a cross section. Both pieces were immersed in 10 % formalin fixing solution (Wako Pure Chemical Industries Ltd.) to fix at room temperature for 24 hours, followed by being equilibrated with 70% ethanol. Subsequently the pieces was dehydrated with ethanol, cleared with xylene, and embedded with paraffin.

Pathohistological examination

Sirius red staining

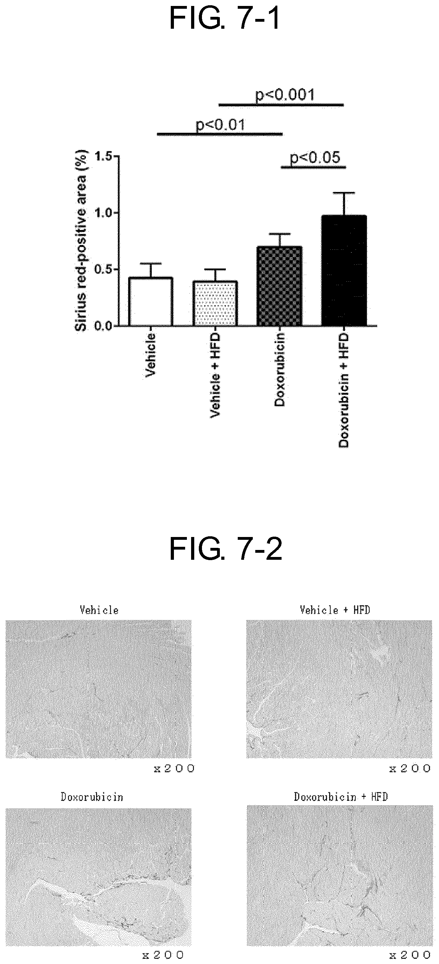

A paraffin section was deparaffinized and hydrophilized, and then, the section was immersed in 0.03% Picro-Sirius Red solution (Sirius Red: Waldeck) for 60 minutes. After dehydrating and clearing, the section was mounted with Entellan new (Merck KGaA). As a result, a rate of a Sirius Red staining positive area increased significantly and increase of collagen accumulation due to the HFD load was observed in Doxorubicin + HFD group compared with Doxorubicin group (Fig. 7-1, 7-2).

The preparation was photographed using the bright field microscope (Leica Microsystems). A collagen positive area rate (fibrosis area) was calculated based on the photographed images using ImageJ.

Flow of statistical analysis

A test of difference between means of respective groups was carried out using Prime 6 (GraphPad Software Inc.). Combinations of the following (1) to (3):

> (1) Vehicle group vs. Vehicle + HFD group
> (2) Vehicle group vs. Doxorubicin group
> (3) Vehicle group vs. Doxorubicin + HFD group were analyzed using Student t test.

An average in each group was expressed as mean $\pm$ SD, and the test was carried out using a significance level of 5%.

[References]

> 14. Edgley AJ et al, Cardiovasc. Ther., 2012, 30, 1, e30-40
> 15. Hagir B. Suliman et al., J. Clin. Invest., 2007, 117, 12, 3730-3741

Example 5: Development of model animal for hepatic cancer

[Study objective]

In evaluation of anti-tumor drugs, many experimental models have been developed and used for drug efficacy evaluation studies so far. Among many cancer model mice, xenograft models using human cancer cells have been used, but these have a problem that reproducibility of a cancer tissue structure including course of development and microenvironment of cancer is low. On the other hand, chemical carcinogenesis models are thought to be more close to human clinical pathology, but there are problems that there is neither simplicity nor evaluation period.

As a model animal for developing a new therapeutic drug for hepatic cancer this time, a DEN-CCl4 dosing mouse model having no diabetes in its background is investigated (see Reference 16). Also in order to examine a synergistic effect by combination of the load of high fat diet (same as in Example 1), a group of receiving DEN-CCl4 administration and the HFD load was set, and raised for 147 days and sacrificed in the same manner.

[Criteria to be preserved and guidelines to be complied, and the like]

Animal welfare

"The Act on Welfare and Management of Animals"

(Article 105 on October 1, 1973, Article 105 finally revised on August 30, 2011)

"The Guideline for Raising and Keeping of Laboratory Animals"

(The Ministry of Environment Notification No. 88, April 28, 2006)

"Fundamental Guideline for Proper Conduct of Animal Experiment and Related Activities in Academic Research Institutes"

(The Ministry of Education, Culture, Sports, Science and Technology Notification No. 71, June 1, 2006).

The present experiments were approved by the Animal Experiment Committee at Stelic MC (SMC Laboratories, Inc.), and performed according to the Guideline for Management and Welfare of Experimental Animals mandated in the experimental facility (Regulations for Animal Experiments at Stelic MC).

[Experimental materials and methods]

Animals to be used

Test animals

Twenty female 14 day pregnant SPF mice (B6C3F1/S1, Japan SLC Inc.) were purchased, put into quarantine, and habituated and raised. All the animals gave birth spontaneously, and males born in these deliveries were used for producing model animals. The male mice were weaned at 4 weeks of age.

Group constitution

The group constitution is shown in Table 6.

Table 6. Group constitution

| Test groups | Sex | The number of animals |
|---|---|---|
| Normal group | Male | 5 |
| DEN-CCl4 group | Male | 12 |
| DEN-CCl4 + HFD group | Male | 10 |

Production of model mouse

DEN (N-nitrosodiethylamine, Sigma Aldrich Japan) was dissolved in PBS to a concentration of 0.2 mg/mL. DEN at 50 $\mu$L/head (0.01 mg/head) was intraperitoneally administered once to the male at 2 weeks of age. Thereafter, a mother animal nursed the male until weaning, and the male was weaned on 28 $\pm$ 2 days after birth.
CCl4 (carbon tetrachloride, Sigma Aldrich Japan) was dissolved in olive oil (Sigma Aldrich Japan) at a concentration of 5% v/v. CCl4 at 100 $\mu$L/head was intraperitoneally administered twice a week to the male mouse at 8 weeks of age. For PBS group and DEN group, olive oil was administered in the same manner.

Observation of general states, measurement of body weight and measurement of feed intake

During the experimental period, the general states were observed, the body weight was measured, and the feed intake was measured. When a dead animal was found, it was immediately necropsied. For reckoning of an experimental day, the following is applied.
Day of starting administration of CCl4 at 8 weeks of age: 0 day (Day 0).

Observation of general states

This was performed before measuring the body weights. The general states such as an outer surface of a body, a nutrient state, an attitude, behaviors and excretions were observed for all of the individuals.

Necropsy

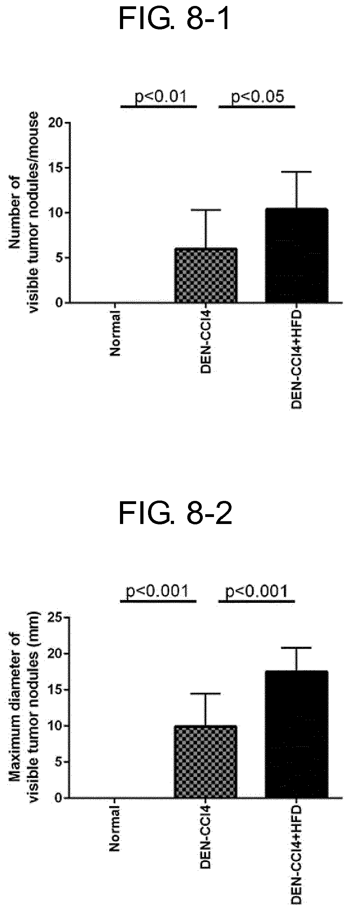

On Day 147, all animals to be necropsied were anesthetized with isoflurane, and blood was collected by heart puncture using heparin as an anti-coagulant. After collecting the blood, liver was harvested. For the liver, the number and a diameter of tumors and nodes that had a maximum diameter of 2 mm or more on a hepatic surface were grossly measured and recorded. As a result, tumor sizes and tumor counts increased significantly and promotion of carcinogenesis due to the HFD load was observed in DEN-CCl4 + HFD group compared with DEN-CCl4 group (Fig. 8-1, 8-2).

A lateral left lobe of the harvested liver was divided into six equal parts. Two parts were immersed in Bouin fixing solution (Sigma Aldrich Japan), fixed at room temperature for 24 hours, and subsequently embedded with paraffin to make a paraffin block. The other two parts were embedded in a CRYO DISH (SHOEI WORK'S Co., Ltd.) filled with OTC compound (Sakura Finetek Co., Ltd.), and immediately frozen in liquid nitrogen to store at -80°C. The other liver parts were frozen and stored.

Sirius red Staining

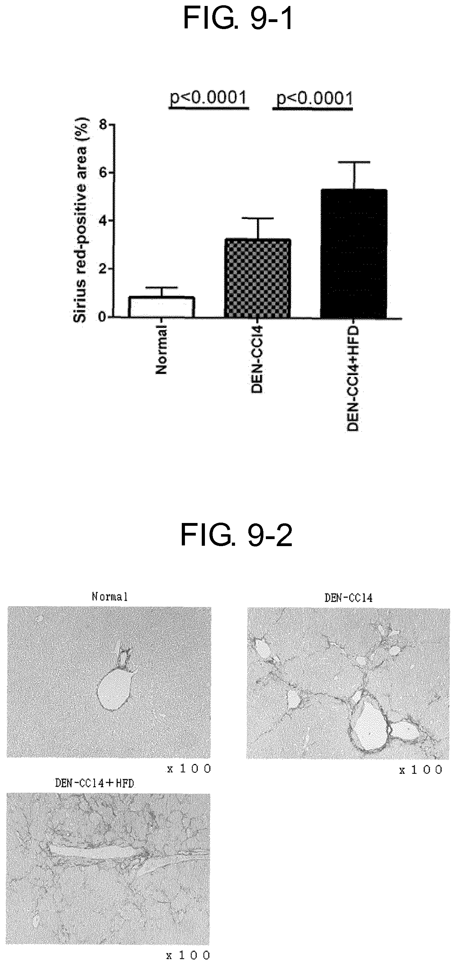

A paraffin section was deparaffinized and hydrophilized, and then, the section was immersed in 0.03% Picro-Sirius Red solution (Sirius Red: Waldeck) for 60 minutes. After passing through 0.5% acetic acid solution and RO water, the section was mounted with Entellan new (Merck KGaA). The preparation was observed using the bright field microscope (Leica Microsystems). Photographs of five fields per one part in the field at 100 times were taken using the CCD camera (Leica Microsystems). A taken area in each field and a Sirius red staining positive area were measured based on the taken images using ImageJ software (National Institute of Health). As a result, a rate of a Sirius red staining positive area increased significantly and increase of collagen accumulation due to the HFD load was observed in DEN-CCl4 + HFD group compared with DEN-CCl4 group (Fig. 9-1, 9-2).

p53 immunostaining

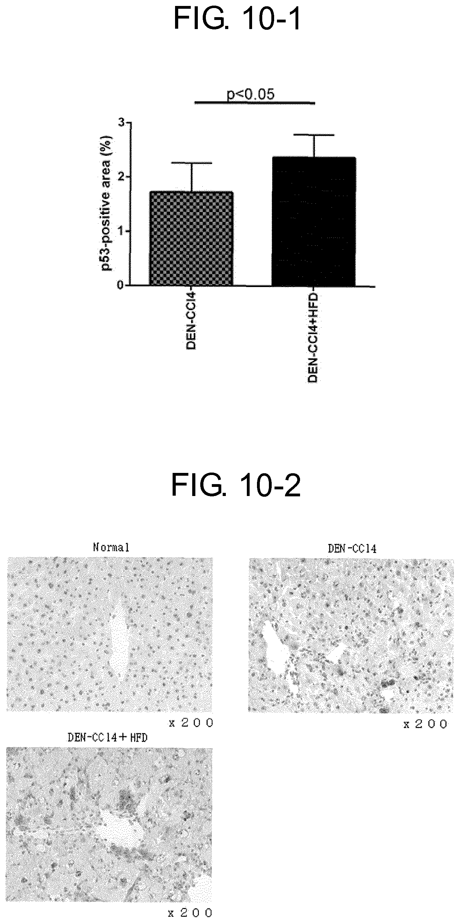

A frozen section made from the frozen block was fixed with acetone, and subsequently endogenous peroxidase was inhibited by 0.03% hydrogen peroxide in water. The section was reacted with a blocking solution (Blockace, DS Pharmabiomedical) at ambient temperature for 10 minutes, and then reacted with an anti p53 antibody (Abcam) diluted 100 times at 4°C overnight. After washing with PBS, the section was reacted with a peroxidase-labeled anti-rabbit IgG goat antibody (Vector) diluted 25 times at ambient temperature for 30 minutes. A color was developed by a chromogenic substrate (Simple stain DAB, Nichirei Bioscience), and the section was mounted with Aquatex (Merck KGaA). The preparation was observed using the bright field microscope (Leica Microsystems). Photographs of five fields per one part in the field of 200 times centered on a central vein were taken using the CCD camera (Leica Microsystems). A taken area of each field and a p53 positive area were measured based on the taken images using ImageJ software (National Institute of Health). As a result, a rate of a p53 immunostaining positive area increased significantly and increase of p53 protein accumulation due to the HFD load was observed in DEN-CCl4 + HFD group compared with DEN-CCl4 group (Fig. 10-1, 10-2). No p53 positive region was detected in the liver section from the normal mouse.

Flow of statistical analysis

Tests of difference between means in respective groups were carried out by Student t-test using Prism 6 (GraphPad Software Inc.) to compare between the normal group and DEN-CCl4 group and between DEN-CCl4 group and DEN-CCl4 + HFD group.

A representative value in each group was expressed as mean $\pm$ SD, and the test was carried out using a significance level of 5%.

[References]

16. The DEN and CCl4-model of fibrosis and inflammation-associated hepatocellular carcinoma, Takeki Uehara, et al, Curr. Protoc. Pharmacol,_2015, 30_1-10

INDUSTRIAL APPLICABILITY

The non-human animal of the present invention that exhibits the protein aggregative lesion is useful for screening of the preventive or therapeutic drug for the disease and evaluation of side effects of the drug.

**Claims**

1. A non-human animal that exhibits a degenerative symptom attributed to protein aggregation, wherein the degenerative symptom attributed to the protein aggregation is induced by misfolding of the protein and said degenerative symptom is promoted.

2. The non-human animal according to claim 1, wherein the degenerative symptom attributed to the protein aggregation is induced by a chemical substance having cytotoxicity.

3. The non-human animal according to claim 2, wherein the chemical substance having the cytotoxicity is (1) combination of diethyl nitrosamine and carbon tetrachloride, (2) 2-amino-1-methyl-6-phenyl-1H-imidazo[4,5-b]pyridine, (3) streptozotocin, (4) adriamycin, or (5) doxorubicin.

4. The non-human animal according to any one of claims 1 to 3, wherein said degenerative symptom is promoted by inducing generation of lipid peroxide.

5. The non-human animal according to claim 4, wherein the generation of said lipid peroxide is induced by feeding of a high fat diet.

6. The non-human animal according to any one of claims 1 to 5, wherein said non-human animal is a model animal of the degenerative disease attributed to the protein aggregation selected from the group consisting of (1) hepatic cancer, (2) Parkinson's disease, (3) Alzheimer's disease, (4) chronic kidney disease, and (5) cardiac fibrosis.

7. The non-human animal according to any one of claims 1 to 6, wherein said non-human animal is a model animal of the degenerative disease attributed to the protein aggregation selected from the group consisting of the following:

(1) a model animal of hepatic cancer produced by a combination of diethyl nitrosamine and carbon tetrachloride, and feeding of the high fat diet;
(2) a model animal of Parkinson's disease produced by 2-amino-1-methyl-6-phenyl-1H-imidazo[4,5-b]pyridine and feeding of the high fat diet;
(3) a model animal of Alzheimer's disease produced by streptozotocin and feeding of the high fat diet;
(4) a model animal of chronic kidney disease produced by adriamycin and feeding of the high fat diet; and
(5) a model animal of cardiac fibrosis produced by doxorubicin and feeding of the high fat diet.

8. The non-human animal according to any one of claims 1 to 7, wherein said non-human animal is a rodent.

9. The non-human animal according to claim 8, wherein the rodent is a mouse.

10. A method for producing a non-human animal that exhibits a degenerative symptom attributed to protein aggregation, comprising the following (1) and (2):

(1) inducing misfolding of a protein to cause the degenerative symptom attributed to the protein aggregation in the non-human animal, and
(2) giving a treatment to promote the degenerative symptom attributed to the protein aggregation in the non-human animal.

11. A non-human animal that exhibits a degenerative symptom attributed to protein aggregation, and to which 2-amino-1-methyl-6-phenyl-1H-imidazo[4,5-b]pyridine is administered.

12. A method for producing a non-human animal that exhibits a degenerative symptom attributed to protein aggregation, comprising administering 2-amino-1-methyl-6-phenyl-1H-imidazo[4,5-b]pyridine.

13. A method for screening of a preventive or therapeutic drug for a protein aggregation disease, comprising the following (a) to (c):

(a) administering a test substance to the non-human animal according to any one of claims 1 to 9 and 11;
(b) evaluating whether the test substance has or not an action of improving a degenerative symptom attributed to protein aggregation; and
(c) selecting the test substance having the action of improving the degenerative symptom attributed to the protein aggregation as the preventive or therapeutic drug for the protein aggregation disease.

14. A method for evaluating a side effect of a test medicine comprising the following (a) and (b):

(a) administrating the test medicine to the non-human animal according to any one of claims 1 to 9 and 11; and
(b) evaluating whether the test medicine has or not an action upon a degenerative symptom attributed to protein aggregation.

15. A biological sample prepared from the non-human animal according to any one of claims 1 to 9 and 11 and including a degenerated site attributed to protein aggregation.

# FIG. 1-1

# FIG. 1-2

# FIG. 2

Vehilce

PhIP

x 400

x 400

P h IP + HFD

x 400

# FIG. 3

# FIG. 4

Sham-control

icv-STZ

x 4 0 0

x 4 0 0

icv-STZ + HFD

x 4 0 0

# FIG. 5-1

# FIG. 5-2

# FIG. 6-1

# FIG. 6-2

# FIG. 7-1

# FIG. 7-2

# FIG. 8-1

# FIG. 8-2

# FIG. 9-1

# FIG. 9-2

# FIG. 10-1

# FIG. 10-2

## INTERNATIONAL SEARCH REPORT

| | International application No. |
|---|---|
| | PCT/JP2017/007955 |

**A. CLASSIFICATION OF SUBJECT MATTER**

*A01K67/027*(2006.01)i, *A61K45/00*(2006.01)i, *A61P25/28*(2006.01)i, *G01N33/15*(2006.01)i, *G01N33/50*(2006.01)i, *C12N15/09*(2006.01)n

According to International Patent Classification (IPC) or to both national classification and IPC

**B. FIELDS SEARCHED**

Minimum documentation searched (classification system followed by classification symbols)
A01K67/027, A61K45/00, A61P25/28, G01N33/15, G01N33/50, C12N15/09

Documentation searched other than minimum documentation to the extent that such documents are included in the fields searched
Jitsuyo Shinan Koho        1922–1996   Jitsuyo Shinan Toroku Koho   1996–2017
Kokai Jitsuyo Shinan Koho  1971–2017   Toroku Jitsuyo Shinan Koho   1994–2017

Electronic data base consulted during the international search (name of data base and, where practicable, search terms used)
JSTPlus/JMEDPlus/JST7580(JDreamIII),
CAplus/REGISTRY/MEDLINE/EMBASE/BIOSIS(STN), WPIDS/WPIX(STN)

**C. DOCUMENTS CONSIDERED TO BE RELEVANT**

| Category* | Citation of document, with indication, where appropriate, of the relevant passages | Relevant to claim No. |
|---|---|---|
| X | UEHARA, T., et. al., UNIT 14.30 The DEN and CCl4-Induced Mouse Model of Fibrosis and Inflammation-Associated Hepatocellular Carcinoma, Current Protocols in Pharmacology, 2014.09, Supplement 66, 14.30.1-14.30.10, ABSTRACT, Prepare the animals | 1-10,13-15 |

☒ Further documents are listed in the continuation of Box C.   ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 08 May 2017 (08.05.17) | 23 May 2017 (23.05.17) |

| Name and mailing address of the ISA/ | Authorized officer |
|---|---|
| Japan Patent Office 3-4-3,Kasumigaseki,Chiyoda-ku, Tokyo 100-8915,Japan | |
| | Telephone No. |

Form PCT/ISA/210 (second sheet) (January 2015)

## INTERNATIONAL SEARCH REPORT

| International application No. |
|---|
| PCT/JP2017/007955 |

C (Continuation). DOCUMENTS CONSIDERED TO BE RELEVANT

| Category* | Citation of document, with indication, where appropriate, of the relevant passages | Relevant to claim No. |
|---|---|---|
| X | TEUNISSEN. S. F., et. al., Development and validation of a liquid chromatography-tandem mass spectrometry assay for the analysis of 2-amino-1-methyl-6-phenylimidazo[4,5-b]pyridine (PhIP) and its metabolite 2-hydroxyamino-1-methyl-6-phenylimidazo[4,5-b]pyridine (N-OH-PhIP) in plasma, urine, bile, intestinal contents, faeces and eight selected tissues from mice., Journal of Chromatography. B, Analytical Technologies in the Biomedical and Life Sciences, 2010, Vol. 878, p. 2353-2362, ABSTRACT, Experimental | 1-6,10-15 |
| X | SHIE, F-S., et al., Obesity and Hepatic Steatosis Are Associated with Elevated Serum Amyloid Beta in Metabolically Stressed APPswe/PS1dE9 Mice, PLoS One, 2015.08.05, 10(8): e0134531, https://doi.org/10.1371/journal.pone.0134531, Abstract, Introduction | 1-10,13-15 |
| X | MITRA, M. S., et.al., High fat diet-fed obese rats are highly sensitive to doxorubicin-induced cardiotoxicity, Toxicology and Applied Pharmacology, 2008, Vol. 231, p. 413-422, ABSTRACT, Materials and methods | 1-10,13-15 |

Form PCT/ISA/210 (continuation of second sheet) (January 2015)

# EP 3 424 316 A1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **GRIGGS A. M. et al.** *Toxicological Science,* 2014, vol. 140 (1), 179-189
- **SINGH,BIRDAVINDER et al.** *Journal of Renin-Angiotensin-Aldosterone System,* 2013, vol. 14 (2), 124-136
- **XUEMING ZHANG et al.** Resolvin D1 Protects Podocytes in Adriamycin-induced Nephropathy through Modulation of 14-3-3β Acetylation. *Plos one,* 2013, vol. 8, 1-17
- **HAGIR B. SULIMAN et al.** *J Clin Invest.,* 2007, vol. 117 (12), 3730-3741
- **PAHWA R. et al.** *Current medical research and opinion,* 2009, vol. 25 (4), 841-849
- **BLESA J. et al.** *NioMed Research Internatinal,* 2012, 2012
- **TEUNISSEN S.F. et al.** *Journal of Chromatography B,* 2010, vol. 878 (25), 2353-2362
- **KAMBOH, M. I. et al.** Genome-wide association study of Alzheimer's disease. *Translational psychiatry,* 2012, vol. 2.5, e117
- **ARVANITAKIS, ZOE et al.** Diabetes mellitus and risk of Alzheimer disease and decline in cognitive function. *Archives of neurology,* 2004, vol. 61 (5), 661-666
- **MEHAN, SIDHARTH et al.** Dementia-A Complete Literature Review on Various Mechanisms Involves in Pathogenesis and an Intracerebroventricular Streptozotocin Induced Alzheimer's Disease. INTECH Open Access Publisher, 2012
- **GORRIE, GEORGE H. et al.** Dendritic spinopathy in transgenic mice expressing ALS/dementia-linked mutant UBQLN2. *Proceedings of the National Academy of Sciences,* 2014, vol. 111 (40), 14524-14529
- Guide for CKD diagnosis 2009. Tokyo Igakusha
- **JUNJI TAKAYAMA.** A simple method for producing a model for reduction of renal functions in rats and mice. *Folia Pharmacol. Jpn.,* 2008, vol. 131, 37-42
- **HAI-CHUN YANG et al.** Models of chronic kidney disease. *Drug discov Today Dis models,* 2010, vol. 7, 13-19
- **LI-JUN MA et al.** Model of robust induction glomerulosclerosis in mice: Importance of genetic background. *Kidney International,* 2003, vol. 64, 350-355
- **XUEMING ZHANG et al.** Resolvin D1 Protects Podocytes in Adriamycin-induced Nephropathy through Modulation of 14-4-4beta Acetylation. *Plos one,* 2013, vol. 8, 1-17
- **EDGLEY AJ et al.** *Cardiovasc. Ther.,* 2012, vol. 30 (1), e30-40
- **HAGIR B. SULIMAN et al.** *J. Clin. Invest.,* 2007, vol. 117 (12), 3730-3741
- **TAKEKI UEHARA et al.** The DEN and CCl4-model of fibrosis and inflammation-associated hepatocellular carcinoma. *Curr. Protoc. Pharmacol,* 2015, vol. 30, 1-10